# FASCICULE DE BREVET EUROPEEN

(11) **EP 2 629 752 B1**
(45) Date de publication et mention de la délivrance du brevet: **13.03.2019**
(21) Numéro de dépôt: 11785741.7
(22) Date de dépôt: 21.10.2011
(51) Int. Cl.: A61K 36/232, A61K 8/97, A61Q 19/08

(54) **COMPOSITION COSMETIQUE OU DERMATOLOGIQUE COMPRENANT UN EXTRAIT D'ANGELIQUE ET SON UTILISATION POUR L'HYDRATATION ET L'ECLAT**
KOSMETISCHE ODER DERMATOLOGISCHE ZUSAMMENSETZUNG MIT EINEM ANGELICA-EXTRAKT UND IHRE VERWENDUNG FÜR FEUCHTIGKEITSVERSORGUNG UND EINEN STRAHLENDEN TEINT
COSMETIC OR DERMATOLOGICAL COMPOSITION INCLUDING AN ANGELICA EXTRACT, AND USE THEREOF FOR MOISTURISATION AND RADIANCE

(30) Priorité: 21.10.2010 FR 1058593
(43) Date de publication de la demande: 28.08.2013
(73) Titulaire: Laboratoires M & L, 04100 Manosque (FR)
(72) Inventeur: DEVILARD, Elisabeth, F-04130 Volx (FR); TOUREL, Cécile, F-130100 Aix en Provence (FR); PIERRISNARD, Jean-Louis, F-04700 Oraison (FR)
(74) Mandataire: Novagraaf Technologies
(86) Numéro de dépôt international: PCT/FR2011/052466
(87) Numéro de publication internationale: WO 2012/052695

(56) Documents cités:
- EP-A2- 2 082 730
- JP-A- 2000 319 155
- JP-A- 2003 171 225
- DATABASE WPI Week 200115 Thomson Scientific, London, GB; AN 2001-141648 XP002639399, "Matrix metalloprotease inhibitor useful in skin external preparation for preventing aging, comprises extracts of a specific genus such as Angelica, Gardenia, Sanguisorba, or lipoic acid or its salt", & JP 2000 319155 A (NOEVIR KK) 21 novembre 2000 (2000-11-21)
- DATABASE WPI Week 200739 Thomson Scientific, London, GB; AN 2007-404823 XP002639400, "Chinese angelica nutrient cream and its preparing method", & CN 1 891 207 A (TANG X) 10 janvier 2007 (2007-01-10)
- DATABASE WPI Week 201023 Thomson Scientific, London, GB; AN 2010-C07365 XP002639405, & KR 2010 018 278 A (AMOREPACIFIC CORP) 17 février 2010 (2010-02-17)
- Anonymous: "Huile Fondante Angélique Bio Peaux Sèches - Naturetis", Herbofée: Produits peaux sèches , 13 février 2009 (2009-02-13), page 1, XP002639401, Extrait de l'Internet: URL:http://herbofee.nexenservices.com/prod uct_info.php?products_id=527&language=fr&p age=5 [extrait le 2011-05-20]
- Anonymos: "Aroma Night - Baume de Nuit nourrissant Angélique", Sefora: Hydratant et Nourrissant , 8 octobre 2009 (2009-10-08), page 1, XP002639402, Extrait de l'Internet: URL:http://avis.sephora.fr/3232-fr_fr/P182 8/reviews.htm [extrait le 2011-05-30]
- Anonymous: "Lancaster 365 Cellular Elixir Delicate Skin", dooyoo: Hautpflege , 15 septembre 2010 (2010-09-15), pages 1-3, XP002639403, Extrait de l'Internet: URL:http://www.dooyoo.de/hautpflege/lancas ter-365-cellular-elixir-delicate-skin/ [extrait le 2011-05-20]
- Anonymous: "PAUL PENDERS Hydrating Control Serum", E-Kosmetica: Paul Penders Products , 24 janvier 2010 (2010-01-24), pages 1-2, XP002639404, Extrait de l'Internet: URL:http://www.e-kosmetika.lv/product_info .php/paul-penders-hydrating-control-serum- p-4127?language=en&cPath=833 [extrait le 2011-05-20]

## Description

### Domaine technique

La présente invention se rapporte à une composition cosmétique ou dermatologique anti-âge, ainsi qu'à une trousse de soin comprenant la composition de l'invention.

La présente invention permet de répondre parfaitement aux besoins quotidiens de la peau afin d'entretenir son éclat et sa beauté, en améliorant son hydratation tout en assurant lissage, protection et prévention des premiers signes de l'âge.

La présente invention se rapporte également à une méthode de traitement cosmétique anti-âge.

Dans la description ci-dessous, les références entre crochets ([]) renvoient à la liste des références présentées à la fin du texte.

### Etat de la technique

Plus qu'une enveloppe extérieure, la peau est un organe à part entière qui joue un rôle essentiel, non seulement dans la protection du corps contre les agressions extérieures, mais aussi sur le plan esthétique et émotionnel. La peau respire, elle est sensible aux agressions extérieures et elle réagit à notre état de santé général ainsi qu'à l'équilibre hormonal.

Une peau en bonne santé est une peau bien hydratée. Elle est douce, ferme, souple et élastique. Le derme est constitué d'une structure cellulaire dont la mission principale est d'assurer l'hydratation de notre peau. Dans l'épiderme, des lamelles de cellules cornées, composées de kératine protectrice en forme de mille feuilles, servent de barrage pour retenir l'eau et filtrer son évaporation. La peau est constituée d'environ 70% d'eau, soit 1/5 de l'eau stockée dans notre corps. L'eau circule du derme vers l'épiderme. Un coussin cellulaire constitué de corps gras à base de lipides, et plus particulièrement de céramides, empêche l'eau de s'évaporer. C'est lui qui détermine le facteur naturel d'hydratation de notre épiderme. Une peau bien hydratée a un taux normal d'hydratation situé entre 13% et 15%. Lorsque ce taux descend à 10%, la peau est déshydratée. Lorsque l'épiderme n'est pas correctement hydraté, par manque d'eau ou d'acides gras essentiels, la peau perd de sa souplesse et devient terne.

L'éclat de la peau est le signe de la bonne oxygénation de ses tissus cutanés. S'il n'y a plus assez d'eau dans le derme, les échanges se font moins bien entre les différentes couches cutanées. Résultat : les fibres de collagène et d'élastine se rigidifient. Les kératinocytes, cellules épidermiques garantes de l'intégrité de la barrière cutanée, s'organisent moins bien. Le bon renouvellement de l'épiderme est compromis.

L'angélique ou Angelica archangelica est d'usage alimentaire, condimentaire et médicinale. Toute la plante est très aromatique. Cultivée dans les monastères d'Europe centrale au XIVe siècle notamment en raison d'une tradition populaire assurant que l'archange Raphael avait révélé à un ermite que l'angélique était un remède contre la peste.

Son nom d'Angelica provient du latin *angelus,* ange et entend souligner l'origine céleste de son parrainage et les propriétés surnaturelles qui avaient été attribuées à l'une des espèces d'angélique, l'Angelica archangelica, l'angélique aromatique [1]. Parfois portée en amulette protectrice, elle a quasiment été considérée comme une panacée au Moyen-âge. A l'époque où l'on voyait dans les crises d'épilepsie le signe d'une possession diabolique, cette plante qui a une action positive sur ce genre d'affection, est aussi dénommée « herbe du Saint-Esprit » [2].

L'angélique officinale est une très grande plante bisannuelle de la famille des Apiacees (ombellifères), elle atteint plus de 2 m. Les feuilles et les tiges se récoltent au début de l'été, les graines, à la fin de l'été (dés qu'elles sont mûres). Les graines doivent être ressemées dès leur maturité, leur capacité germinative étant réduite à quelques semaines. Les racines sont ramassées a la fin de l'automne, en deuxième ou troisième année de croissance. Angelica archangelica est une espèce non spontanée en France. (Rameau 1989).

Le genre Angelica comprend en France trois espèces sauvages :
- L'Angélique sauvage, Angelica sylvestris, présente dans toute la France, rare dans le midi ;
- L'Angélique des estuaires, Angelica heterocarpa, présente sur le littoral atlantique sud, espèce protégée au niveau national (arrêté de 1982) ; et
- L'Angélique de Razouls, Angelica razulii, présente dans toute la chaîne des Pyrénées.

L'Angélique, ombellifère géante, était en effet supposée conjurer les envoûtements. Accrochée au cou des enfants, elle protégeait en particulier ces derniers contre les maléfices de toute nature. Connue et utilisée au Moyen-âge dans le nord et le centre de l'Europe, elle ne gagna nos régions qu'au XVe siècle.

Les médecins de la Renaissance surnommaient sa racine " Racine du Saint-Esprit ", à cause de ses " grandes et diverses propriétés contre de très-griefves maladies ". Ainsi Paracelse (1490-1541) rapporte-t-il que, lors des grandes épidémies de peste de 1510, de nombreux Milanais furent sauvés grâce à ses prescriptions : de l'Angélique en poudre dissoute dans du vin.□Dès le XIVème siècle, on la cultivait dans les monastères de l'Europe centrale sous le nom d'Herbe-du-Saint-Esprit, dès le XVIème siècle, sa culture était répandue un peu partout, et l'on sélectionnait les races à racines plus développées. Jusqu'au XVIIIème siècle, l'usage médical l'emportait beaucoup sur l'emploi en liquoristerie [3]. En 1629, Parkinson dans son traité « Paradise in Sole » avait affirmé la suprématie de l'Angélique sur toutes les autres plantes médicinales [4]. diverses propriétés médicinales anciennement reconnues. Tonique, stimulante, stomachique, sudorifique, emménagogue, carminative...:" l'herbe aux anges " pourrait concurrencer le " ginseng " chinois.

Une publication basée sur des études scientifiques présentée dans Curr Med Chem [5] dresse un résumé complet des nombreuses et diverses propriétés biologiques de l'Angélique : antitumorale [6], hépatoprotectrice [7] néphroprotectrice, anti-inflammatoire, analgésique, antibactérienne...

JP2003-171225 divulgue qu'un extrait alcoolique de racine d'angélique stimule la production d'intégrine dans l'épiderme et protège la peau des rayonnements UV.

Plus généralement, il est admis que sa racine est un excellent tonique de l'état général, contre la fatigue, l'asthénie, bénéfique pour tout le système digestif. En effet, elle contient une huile essentielle très riche en terpènes conférant les propriétés hépato et gastroprotectrices : indiquée en cas de douleurs et spasmes intestinaux, dyspepsies (mauvaises digestions). L'angélique évite la formation de gaz intestinaux qui peuvent provoquer des problèmes de ballonnements et d'aérophagie.

Outre ses vertus carminatives, l'huile essentielle a également des propriétés sédatives, particulièrement indiquée en cas d'anxiété, de fatigue nerveuse, d'insomnie et de troubles du sommeil. Enfin, l'huile essentielle est également citée pour ses propriétés anticoagulantes.

Par ailleurs, les chercheurs ont découvert dans l'HE d'Angélique des propriétés fongicides et antibactériennes grâce à la présence d'un macrolide présent dans les graines et les racines [8].

En Europe on l'utilise surtout pour traiter les refroidissements, les bronchites, la toux, les troubles urinaires et l'indigestion [4].

Les vertus purifiantes et tonifiantes de l'Angélique permettent d'obtenir une excellente eau de toilette et un bon élixir dentifrice.

L'angélique est utilisée également en parfumerie et à des fins culinaires où elle est toujours très consommée, crue ou cuite, dans le Nord de l'Europe (Islande, Laponie, Norvège, Sibérie).

Par ailleurs, les pétioles des feuilles et les jeunes tiges sont confites et utilisées en pâtisserie sous forme de fruits confits. Cette activité a généré de grandes surfaces cultivées principalement en Auvergne et dans les Deux-Sèvres. L'angélique confite est la spécialité de la ville de Niort depuis le XVIIIème siècle.

### Exposé de l'invention

La présente invention a précisément pour but de répondre à ce besoin en fournissant une composition cosmétique et/ou dermatologique comprenant un extrait d'Angélique en tant que principe actif anti-âge.

En d'autres termes, la présente invention se rapporte à l'utilisation cosmétique et/ou dermatologique d'un extrait d'Angélique en tant que principe actif anti-âge.

Les inventeurs à l'origine de la présente sont en effet les tout premiers à avoir mis en évidence, de manière tout à fait inattendue, qu'un extrait d'Angélique permet de manière cumulée la stimulation de l'aquaporine 3 (AQP3), la stimulation de la prolifération des kératinocytes, le piégeage des radicaux libres, la stimulation de la synthèse des glycosaminoglycanes et l'inhibition des métalloprotéases matricielles (MMPs) de la peau. Les expérimentations présentées ci-dessous démontrent largement ces effets.

Grâce à ces actions nombreuses et cumulées, l'utilisation cosmétique et/ou dermatologique d'un extrait d'Angélique conforme à la présente invention permet à la fois de renforcer l'hydratation naturelle de la peau, d'améliorer son élasticité et de la protéger des radicaux libres pour un résultat rebondi, lisse et éclatant. La composition de la présente invention permet de préserver une peau éclatante. Les extraits agissent de façon synergique sur l'hydratation, le renouvellement cellulaire, l'élasticité et la protection de la peau.

Selon l'invention, l'extrait d'Angélique est un mélange d'un extrait aqueux et d'un extrait huileux.

Selon l'invention, pour ce qui est de l'extrait aqueux, il peut s'agir de préférence d'une eau d'Angélique, par exemple Végébios d'angélique (marque de commerce) de la société Solabia (France) obtenu par extraction aqueuse.

Selon l'invention, l'extrait aqueux d'Angélique peut être présent dans la composition par exemple à une concentration de 0,01 à 10% en poids par rapport au poids total de ladite composition, par exemple de 0,01 à 5% en poids par rapport au poids total de ladite composition. L'extrait aqueux d'Angélique permet notamment de stimuler les aquaporines, en particulier l'amélioration de la circulation de l'eau vers les cellules de la couche cornée, et favorise le renouvellement cellulaire.

Selon l'invention, en ce qui concerne l'extrait huileux, il peut s'agir de préférence d'une huile essentielle de racine d'Angélique, par exemple l'huile essentielle d'angélique des Laboratoires Rosier Davenne (France) obtenu par distillation à la vapeur d'eau.

Selon l'invention, l'extrait huileux d'Angélique peut être présent dans la composition par exemple à une concentration de 0,001 à 0,8% en poids par rapport au poids total de ladite composition, par exemple de 0,001 à 0,5% en poids par rapport au poids total de ladite composition. L'extrait huileux permet notamment de préserver l'élastine, de protéger des radicaux libres et de stimuler la synthèse des GAGs.

Quel que soit le mélange d'extraits, selon l'invention, la composition peut comprendre en outre d'autres extraits de plante, par exemple un extrait de pensée sauvage.

Selon l'invention, la composition de la présente invention peut comprendre en outre un sucre, par exemple le D-glucoside de xylityle. Ce sucre particulier, commercialisé par la société SEPPIC (France), augmente la teneur hydrique de l'épiderme et les réservoirs hydriques dermiques par augmentation de la synthèse des glycosaminoglycanes (GAGs). Il permet également de limiter les pertes hydriques en renforçant la barrière cutanée par augmentation de la synthèse des Céramides et en limitant les pertes d'eau.

Selon l'invention, quel que soit le mélange d'extraits utilisés, la composition cosmétique ou dermatologique de la présente invention peut comprendre en outre au moins un agent actif additionnel choisi parmi un agent anti-âge, un agent hydratant, un agent apaisant ou un mélange de ces agents. Ces agents peuvent être ceux couramment utilisés dans les produits cosmétiques et/ou dermatologiques. Des exemples d'agents anti-âge utilisables sont par exemple le silicium ou la vitamine C. Des exemples d'agents hydratants utilisables sont par exemple la glycérine ou l'acide hyaluronique. Des exemples d'agents apaisants utilisables sont par exemple le bisabolol ou l'allantoïne. Ce ou ces agent(s) sont utilisables dans la composition de la présente invention par exemple aux concentrations usuellement utilisées et connues de l'homme du métier dans les compositions cosmétiques ou dermatologiques.

Selon l'invention, le mélange d'extraits d'Angélique ou la composition de la présente invention peut être ajoutée à une composition cosmétique existante ou faire l'objet d'une composition propre, c'est à dire développée spécialement pour recevoir la composition de la présente invention.

Selon l'invention, la composition de la présente invention peut se présenter sous toute forme connue de l'homme du métier dans les domaines de la cosmétique et de la dermatologie, sans aucune restriction galénique particulière. Bien entendu, la composition cosmétique doit pouvoir être appliquée sur la peau et être compatible avec l'extrait d'Angélique utilisé selon la présente invention. Selon l'invention, la composition cosmétique peut par exemple être sous une forme choisie dans le groupe comprenant une crème, un lait, une lotion, un gel, un sérum, un onguent. La composition peut par exemple aussi se présenter sous la forme d'un masque.

La présente invention se rapporte également à une trousse comprenant une composition cosmétique selon la présente invention et un manuel ou une notice d'utilisation. La trousse peut être par exemple un conditionnement.

La présente invention se rapporte également à un procédé de soin cosmétique anti-âge comprenant une étape d'application sur la peau d'un mélange d'extraits d'Angélique ou d'une composition cosmétique selon l'invention.

La présente invention se rapporte également à une trousse de soins comprenant une composition selon l'une quelconque des revendications précédentes et un manuel ou une notice d'utilisation. Ce manuel ou cette notice a notamment pour fonction d'expliquer à l'utilisateur la manière et la fréquence d'application sur la peau la composition de la présente invention.

La présente invention se rapporte également à un procédé de traitement cosmétique comprenant une étape d'application d'une composition cosmétique ou dermatologique selon l'invention. Il peut s'agir d'une simple application ou d'une application accompagnée d'un massage de la peau avec la composition de la présente invention. L'application est réalisée avec une quantité suffisante de la composition afin que toute la surface de la peau à traiter soit traitée. Il peut s'agir par exemple d'une application classique, comme une crème sur la peau. Il peut s'agir par exemple aussi d'une application pour former un masque traitant.

D'autres caractéristiques et avantages apparaîtront encore à l'homme du métier à la lecture des exemples ci-dessous, donnés à titre illustratif et non limitatif.

### EXEMPLES

### Exemple 1 : Exemple d'un soin du visage réalisé à partir de la composition de la présente invention

On prépare par mélange des différents composants, une composition de base décrite dans le tableau 1 ci-dessous :

**Tableau 1 : Exemple d'une composition pour le visage**

| **Ingrédients** | **Quantité en % par rapport au poids total de la composition (%)** |
|---|---|
| Stéarate de sucrose | 0,5 à 6% |
| Distéarate de sucrose | 0,5 à 6% |
| Triglycéride caprylique/caprique | 3 à 20% |
| Glycérine | 0,5 à 10% |
| Gomme xanthane | 0,1 à 0,5% |
| Parfum | Qs |
| Conservateurs | Qs |
| Eau | qsp 100% |

A partir de cette composition de base, pour le soin du visage, on fabrique 12 compositions différentes conformes à la présente invention en ajoutant différents extraits en différentes quantités :
- Six compositions comprenant de l'Eau d'angélique avec respectivement : 0,01 ; 0,05 ; 0,1 ; 1 ; 2 et 5% en poids par rapport au poids total de chaque composition ;
- Six composition comprenant de l'Huile essentielle d'angélique avec respectivement : 0,001 ; 0,01 ; 0,02, 0,2 ; 0,5 et 0,8% en poids par rapport au poids total de chaque composition.

Extraits utilisés : Eau d'angélique de Végébios (marque de commerce) de Solabia obtenu par extraction aqueuse et huile essentielle d'angélique des Laboratoires Rosier Davenne obtenue par distillation à la vapeur d'eau.

En plus de ces 12 compositions, on prépare les compositions suivantes :
- 2 lots de 4 compositions dans lesquelles on ajoute du D-glucoside de xylityle à raison de 1,5 ; 2 ; 2,5 ou 3% en poids par rapport au poids total de chaque composition ; un des 2 lots comprenant 0,01% en poids d'extrait aqueux d'angélique par rapport au poids total de la composition et l'autre 5% en poids d'extrait aqueux d'angélique par rapport au poids total de la composition.
- 2 lots de 5 compositions dans lesquels on ajoute un extrait de pensée sauvage à raison de 0,01 ; 0,05 ; 1 ; 2 et 5% en poids par rapport au poids total de la composition ; un des 2 lots comprenant 0,001% en poids d'huile essentielle d'angélique par rapport au poids total de la composition et l'autre 0,8% en poids d'huile essentielle d'angélique par rapport au poids total de la composition.

Le glucoside de xylityle provient de la société SEPPIC, sous la référence commerciale AQUAXYL, l'extrait de pensée sauvage utilisé est Aquaphyline (marque de commerce) de Silab (France) obtenu par hydrolyse enzymatique d'un mélange pensée sauvage/eau.

Les valeurs en parfum, conservateur et eau étant alors ajustées comme indiqué dans le tableau ci-dessus.

### Exemple 2 : Effet d'un hydrolat de racine d'Angélique ou d'un extrait aqueux d'Angélique ou de l'huile essentielle de racine d'Angélique pour hydrater durablement la peau

La peau peut subir toutes sortes d'agressions extérieures (soleil, conditions climatiques, pollution, mauvaise hygiène de vie) entrainant une perte d'hydratation. Le flux trans-épidermique ou perte insensible en eau est le processus issu de la circulation sanguine diffuse via le derme pour s'évaporer en surface. Le flux est régulé par la nature même de la structure cutanée et la couche superficielle de l'épiderme est constituée d'un empilement de cellules plates qui constituent un ciment composé de céramides, d'acides gras et de cholestérol. La capacité de l'épiderme à retenir l'eau est liée à un ensemble de molécules hygroscopiques intracellulaires, le NMF (acides aminés provenant de la dégradation de la filaggrine, acides gras, urée, lactate de sodium). D'autre part, de petites protéines membranaires, véritables canaux à eau, les aquaporines, forment des « pores » perméables aux molécules d'eau dans les membranes biologiques. Les aquaporines permettent le passage de l'eau de part et d'autre de la membrane tout en empêchant les ions de pénétrer dans la cellule. Il existe 13 aquaporines chez l'homme (8) localisées dans les cellules épithéliales avec des spécificités différentes. Au niveau de la peau et plus particulièrement dans l'épiderme, l'aquaporine 3 (AQP3) assure le passage de l'eau.

Les travaux réalisés par la Demanderesse dans le cadre de l'invention ont permis de mettre en évidence, que l'eau d'Angélique stimule de façon significative l'expression et la synthèse de l'aquaporine 3 (AQP3). Ces travaux sont résumés ci-dessous.

### Effet sur la stimulation de l'AQP3 d'un extrait aqueux d'Angélique par immunofluorescence

Les expériences sont réalisées avec de l'Eau d'angélique Végébios de Solabia obtenu par extraction aqueuse

Le but de ces expériences était de valider *in vitro* la stimulation de l'AQP3 par un extrait d'Angélique.

### Matériel et méthode

Des extraits (eau d'Angélique, un hydrolat de racine d'Angélique et l'huile essentielle de racine d'Angélique) aux différentes concentrations présentées dans l'exemple 1 sont réalisés en vue de tester la stimulation de l'expression de l'AQP3.

Après avoir été incubés pendant 24 heures dans du milieu de culture Kératinocytes SFM (sans sérum ou « serum free médium » pour SFM) avec les différents extraits et concentrations, des kératinocytes humains en culture (ATCC, PCS-200-011) sont rincés par du PBS puis fixés en paraformaldéhyde 3% et perméabilisés avec du Triton 0,1%.

Les cellules sont ensuite incubées à température ambiante (25°C) en présence d'un anticorps anti AQP3 (goat polyclonal IgG, TEBU/ réf. SC-9885) dilué au 1/50 dans du PBS pendant 1 heure. Après lavage dans du PBS, un anticorps secondaire RAG-Alexa 568 (rabbit anti-goat, Fisher/ ref.W3009H) dilué au 1/200^{e} dans du PBS est rajouté et incubé pendant 1H à température ambiante et à l'obscurité. Après lavage par du PBS.

Une contre coloration est réalisée afin de faciliter l'interprétation des résultats en marquant le noyau des kératinocytes par une solution de Hoechst 332 (référence commerciale), celui-ci se fixant spécifiquement sur les bases de l'ADN (A, T).

Une acquisition des images est réalisée avec le INCell Analyzer 1000 (marque commerciale) (GE Healthcare) permettant de mesurer l'intensité de la fluorescence et ainsi de quantifier l'expression de AQP3 dans les kératinocytes.

### Résultats

Les résultats sont regroupés et visualisés dans le tableau 2 ci dessous exprimé en % de stimulation de l'AQP3. Un test de Student (t-test) a été également réalisé permettant de donner une valeur statistique à ces mesures. Ce test paramétrique compare la moyenne observée d'un échantillon statistique à une valeur fixée, ou encore la probabilité observée d'un caractère à une probabilité théorique. Il permet aussi de comparer les moyennes de deux échantillons statistiques, on parle alors de test de conformité.

**Tableau 2 : expression en % de la stimulation de l'AQP3 et t-test**

| | **eau d'Angélique** | **hydrolat racine Angélique** | **HE Angélique** |
|---|---|---|---|
| **Concentrations testées en % en poids par rapport au poids de la composition** | 0,2 | 0,04 | 5,5.10⁻⁵ |
| | 1 | 0,2 | 1,7.10⁻⁴ |
| | 5 | 1 | 5.10⁻⁴ |
| **% STIMULATION DES AQP3** | 0 | 0 | 0 |
| | 39 | 0 | 0 |
| | 19 | 0 | 0 |
| ***Valeur P*** | NS | NS | NS |
| | * | NS | NS |
| | ** | NS | NS |

| | | | |
|---|---|---|---|
| Valeur du t-test NS : > 0.05, non significatif *: 0,01 to 0,05, significatif **: 0,001 to 0,01, très significatif ***: < 0,001, extrêmement significatif | | | |

De ces résultats, on peut conclure que l'eau d'angélique entraine la stimulation de l'aquaporine 3 de façon très significative. L'eau d'Angélique à 2% stimule de + de 19% l'expression de l'AQP3, et ce même extrait à 5% entraine la stimulation de l'expression de l'APQ3 de 39%.

L'eau d'angélique stimule donc l'expression de l'aquaporine 3, très présente dans les kératinocytes de l'épiderme assurant ainsi la circulation de l'eau vers les cellules de la couche cornée et donc une bonne hydratation de la peau en continue.

### Exemple 3 : Effet d'un hydrolat de racine d'Angélique ou d'un extrait aqueux d'Angélique ou de l'huile essentielle de racine d'Angélique pour revitaliser et renouveler la peau

Quand la peau est mal hydratée, les échanges entre derme et épiderme se font moins bien. De plus, les kératinocytes de l'épiderme, garants de l'intégrité de la barrière cutanée, s'organisent moins bien. Leur bon renouvellement et a fortiori celui de l'épiderme est compromis.

Une publication de Hensel et al. « Occurrence of N-phenylpropenoyl-L-amino acid amides in différent herbal drugs and their influence on human keratinocytes, on human liver cells and on adhesion of Helicobacter pylori to the human stomach"; Planta Med. 2007 Feb;73(2):142-50 (10) fait référence à la présence en grande quantité d'amide particulier entrainant une forte prolifération (150%) des kératinocytes humains *in vitro* avec amplification du facteur de transcription STAT6.

A travers le maintient du revêtement épidermique (prolifération des kératinocytes), l'extrait de racine d'Angélique peut contribuer à maintenir une bonne hydratation et surtout rehausser l'éclat de la peau.

Les travaux réalisés par la Demanderesse dans le cadre de l'invention ont permis de mettre en évidence, que l'extrait biologique aqueux d'Angélique entraine, de façon significative, la prolifération des kératinocytes humains.

### Effet d'un extrait d'Angélique aqueux sur la prolifération des kératinocytes humains par le test BrdU

Le but de cette étude est de valider in vitro la prolifération des kératinocytes humains par un extrait aqueux d'Angélique par incorporation de BrdU, analogue de la thymidine, l'une des bases de l'ADN, et permet donc de marquer la prolifération cellulaire. Plus le BrdU est incorporé, plus les cellules prolifèrent.

### Matériel et méthode

Des extraits (extrait aqueux d'Angélique, un hydrolat de racine d'Angélique et l'huile essentielle d'Angélique) à différentes concentrations sont réalisés en vue de tester l'incorporation de BrdU.

Les extraits utilisés sont l'eau d'angélique Végébios de Solabia obtenu par extraction aqueuse, l'hydrolat de racine d'angélique des Laboratoires Rosier Davenne obtenu par condensation lors de la distillation de l'huile essentielle à la vapeur d'eau, et l'huile essentielle d'angélique des Laboratoires Rosier Davenne obtenu par distillation à la vapeur d'eau.

Après avoir été cultivés dans le milieu de culture Kératinocytes SFM médium pendant 24h à 37°C, les kératinocytes sont ensuite incubés à 37°C en présence des différentes concentrations (voir tableau 3) de chaque extrait d'Angélique ou en présence de facteur de croissance épidermique (« Epidermal Growth Factor » (EGF)) à 0,.25 ng/ml + Pituitary Extract (PE) à 25 µg/ml (réfèrent contrôle positif pour le 100% prolifération) ou sans aucun extrait ou autre (contrôle négatif/ 0% de prolifération) pendant 72h.

Puis le BrdU est ajouté et incubé à 37°C pendant 24h supplémentaire.

A la fin de l'incubation, la prolifération des kératinocytes est quantifiée à l'aide d'un kit commercialisé par Roche (BrdU /ref.1.647.229) et l'intensité de la coloration est mesurée à 450nm par un spectrophotomètre.

### Résultats

L'intensité de coloration est proportionnelle à la prolifération. Les résultats sont reproduits dans le tableau 3 ci-dessous. Un test de Student (p-value) a été réalisé afin d'ajouter une valeur statistique à ces mesures.

**Tableau 3 : incorporation de BrdU des différents extraits d'Angélique et test statistique**

| | extrait aqueux Angélique | hydrolat racine Angélique | HE Angélique |
|---|---|---|---|
| concentrations testées en % en rapport au poids de la composition | 0,2 | 0,04 | 5,5x10⁻⁵ |
| | | | |
| | 1 | 0,2 | 1,7x10⁻⁴ |
| | 5 | 1 | 5x10⁻⁴ |
| % de stimulation des kératinocytes | 7 | 1 | 0 |
| | | | |
| | 15 | 0 | 2 |
| | 17 | 1 | 0 |
| Valeur P | NS | NS | NS |
| | ** | NS | NS |
| | *** | NS | NS |

| | | | |
|---|---|---|---|
| **Valeur du t-test** NS : > 0,05, non significatif *: 0,01 to 0,05, significatif **: 0,001 to 0,01, très significatif ***: < 0,001, extrêmement significatif | | | |

De ce fait, on peut conclure que l'extrait aqueux d'Angélique entraine une prolifération des kératinocytes humains très significative à 1% et une prolifération extrêmement significative à 5%

Nous retrouvons ainsi les résultats publiés par Hensel et al. Les amides impliqués dans la prolifération des kératinocytes humains sont contenus en très grande quantité dans les racines.

L'extrait aqueux d'Angélique en entrainant la prolifération des kératinocytes assure ainsi le bon renouvellement de l'épiderme.

### Exemple 4 : Effet d'un extrait aqueux d'Angélique ou de l'huile essentielle de racine d'Angélique afin de rehausser l'éclat de la peau et combattre les premiers signes de l'âge.

Lorsque la peau est mal irriguée, les différents compartiments cellulaires sont moins bien oxygénés ce qui entraine une perte d'éclat de la peau et une modification de l'architecture entre le derme et l'épiderme et ainsi, les premiers signes de l'âge apparaissent.

Les travaux réalisés par le Demanderesse dans le cadre de l'invention ont permis de mettre en évidence, que l'huile essentielle de racine d'angélique raffermit et protège la peau piégeant les radicaux libres, protégeant la matrice extracellulaire de toute dégradation et stimule les GAGs (forment le réseau extra membranaire).

### A/ Effet anti radicalaire de l'huile essentielle d'Angélique ou de l'extrait aqueux d'Angélique

Le but de cette étude est de valider In vitro l'activité anti radicalaire de l'huile essentielle de racine ou extrait aqueux d'Angélique.

### 1. Matériel et méthode

L'huile essentielle et extrait aqueux d'Angélique à différentes concentrations (5-1-0,2-0,04 et 0,008 %) sont réalisés en vue de tester l'action anti radicalaire par le test de réduction du DPPH [11]. En effet, le DDPH (2,2 diphényl-1-picrylhydrasyl) est un radical stable réduit par les antioxydants en 1,1-diphényle-2-picrylhydrazine dont l'intensité, proportionnelle au DDPH réduit, de coloration développée est mesurée au spectrophotomètre.

Extraits utilisés : huile essentielle d'angélique des Laboratoires Rosier Davenne obtenu par distillation à la vapeur d'eau et extrait aqueux d'angélique Végébios de Solabia obtenu par extraction aqueuse

La vitamine E est utilisée comme référent contrôle.

Ces réactions sont réalisées en plaques de 96 puits et répétées 3 fois.

### 2. Résultats

Les résultats obtenus sont récapitulés et visualisés dans le tableau 4 ci-dessous.

**Tableau 4 : test anti radicalaire exprimé en % d'activité et test statistique (p-value)**

| **Concentrations en % en poids par rapport au poids total de la composition** | **extrait aqueux Angélique %inhibition Valeur P** | **HE Angélique %inhibition Valeur P** |
|---|---|---|
| **8.10-3 %** | 0 | 0 |
| | NS | NS |
| **0,04%** | 0 | 0 |
| | NS | NS |
| **0,20%** | 0 | 0,5 |
| | NS | NS |
| **1%** | 0,5 | 3 |
| | NS | NS |
| **5%** | 1 | 10 |
| | NS | ** |

De ce fait, les résultats de ce test montrent que l'huile essentielle de racine d'Angélique réduit l'activité des radicaux libres. En piégeant les radicaux libres, les cellules de la peau sont protégées du vieillissement et la peau reste donc jeune plus longtemps.

### B/ Effet de l'huile essentielle de racine d'Angélique sur la synthèse de GAGs par les kératinocytes humains cultivés en monocouche.

L'objectif de cette étude est de valider in vitro l'action de l'huile essentielle de racine d'Angélique sur la synthèse des GAGs associée ou non à un extrait de pensée sauvage.

L'extrait de pensée sauvage (Aquaphyline de la société SILAB, France), riche en oligosaccharides, améliore l'irrigation cutanée en stimulant, d'une part, la synthèse des aquaporines. D'autre part, Aquaphyline accroit la capacité de rétention d'eau dermique en augmentant l'activité de la hyaluronane synthétase et le taux et la fixation de l'acide hyaluronique dans l'épiderme. Des études in vitro ont été menées par SILAB validant ainsi l'activité de cet extrait de pensée sauvage.

### 1. Matériel et méthode

Dans ce test, les effets des composés HE Angélique racine et AQUAPHYLINE EL (pensée sauvage) sur la néo synthèse des glycosaminoglycanes (GAGs) totaux produits par les kératinocytes humains normaux (NHEK) cultivés en monocouche ont été évalués par mesure de l'incorporation de [3H]-glucosamine dans les GAGs néo synthétisés.

Après culture dans le milieu Keratinocytes SFM médium, les kératinocytes humains sont incubés pendant 72h à 37°C avec les différentes concentrations en extraits et mélangés (voir tableau 5) en présence de CaCl₂ (référent/contrôle positif) ou sans rien (contrôle négatif).

Le traceur radioactif 3H*glucosamide (Perkin Elmer, Ref.NET 190A) à la concentration de 3,33 µCi/puit est ensuite incorporé dans le milieu de culture contenant ou non les différentes concentrations en extraits et mélanges et incubé 24h en plus à 37°C.

A la fin de l'incubation, les GAGs néosynthétisés sont extraits par un tampon particulier composé de Tri/Hcl 50mM, guanidine 4M et EDTA 5mM et purifié par chromatographie échangeuse d'ions. La radioactivité est alors dosée et quantifiée.

### 2. Résultats

Les résultats obtenus sont résumés et visualisés dans le tableau 5 ci-dessous.

**Tableau 5 : stimulation de la production de GAGs par les kératinocytes en % de stimulation**

| **Extraits** | **Concentrations en %** | **% de stimulation** |
|---|---|---|
| **CaCl2/ct+** | **1,5 mM** | **53** |
| HE racine Angélique | **0,0004** | 40 |
| | **0,002** | 29 |
| | **0,01** | 34 |
| Aquaphyline pensée sauvage | **0,0004** | 37 |
| | **0,002** | 36 |
| | **0,01** | 23 |
| HE+Aquaphyline | **0,0004+0,0004** | 72 |
| | **0,002+0,002** | 41 |
| | **0,01+0,01** | 34 |

Le traitement par le calcium, à 1.5 mM, a stimulé significativement la néosynthèse des GAGs totaux (53% de stimulation).

Dans les conditions expérimentales de cette étude, pour l'ensemble des concentrations testées, les 2 composés ainsi que leur mélange ont tous augmenté de façon significative la néosynthèse des GAGs totaux. Par ailleurs, le mélange des 2 composés permet d'observer un effet synergique sur la néosynthèse des GAGs totaux.

L'huile essentielle de racine d'Angélique stimule donc les kératinocytes qui produisent plus de GAGs responsables de la fermeté de la peau avec le collagène. La peau est donc plus dense et se raffermit.

### C/ Effet de l'huile essentielle de racine d'Angélique sur l'inhibition de l'activité des MMPs (dégradation de la matrice extracellulaire)

L'objectif de cette étude est de valider in vitro l'action de l'huile essentielle de racine d'Angélique sur l'inhibition des MMPs. Matériel et méthode.

Afin d'activer les MMPs issues de fibroblastes dermiques normaux en culture, ceux-ci sont irradiés sous UV(20mJ/cm2) puis traités avec un mélange inflammatoire composé de Phorbol Myristate Acetate (PMA) et le Tumor Necrosis TNF Factor alpha (TNF-a).

Les composés de référence, les différentes concentrations d'huile essentielle sont incubées pendant 10 minutes à 4°C en présence des MMPs activés. Le substrat chromogène fluorescent, petit peptide (Mca-Pro-Meu-Gly-Leu-Dpa-Ala-Arg-NH₂) est ensuite rajouté à la concentration de 1/50^{e} et incubé pendant 3h à 37°C.

Les expériences sont réalisées en triplicate, avec contrôle interne intégré. A la fin de l'incubation, la fluorescence produite par la dégradation du substrat est mesurée au spectrophotomètre à 393nm.

### 1. Résultats

Les résultats sont exprimés en % d'inhibition de l'activité des MMPs et présentés dans le tableau 6 suivant :

**Le tableau 6 regroupe tous les résultats ci-dessous**

| **Extraits** | **Concentrations en %** | **% d' inhibition MMPs** |
|---|---|---|
| **O phénathroline** | **1 mM** | **100** |
| HE racine Angélique | **0,04** | -18 |
| | **0,2** | -6 |
| | **1** | **18** |

Le composé de référence O-Phénantroline testé à 1mM, a totalement inhibé l'activité des MMPs totales.

Le composé HE Angélique de racine a présenté un effet inhibiteur spécifique sur l'activité MMPs totales.

On peut conclure que l'Huile Essentielle de racine d'Angélique permet de maintenir l'architecture de la matrice extracellulaire et les échanges entre le derme et l'épiderme en inhibant les MMPs. La peau est ainsi protégée contre les premiers signes de l'âge.

### Exemple 5 : Test d'usage du soin visage anti-âge et évaluation des qualités hydratantes, lissantes, protectrices et éclat de la peau de cette composition

Pour répondre parfaitement et de façon spécifique aux peaux déshydratées avec perte d'éclat, les inventeurs ont mis au point un complexe d'actifs synergiques assurant à la fois un effet hydratant, lissant, protecteur.

De ce fait, la composition ci-dessus (tableau 1) a été appliquée de manière quotidienne pendant 28 jours par 49 femmes dans les conditions normales d'utilisation afin d'évaluer ses qualités cosmétiques. Les caractéristiques des volontaires sont présentées dans le tableau 7 ci-dessous.

**Tableau 7 : caractéristiques des volontaires recrutés pour cette étude**

| **Volontaires** | **Nature de la peau du visage** | **Sensibilité** | **Volontaires Sains à terrain atopique** | **Utilisateurs de ce type de produit** |
|---|---|---|---|---|
| Nombre : 49 | Normale : 4 (8%) | Peau du visage : 20 (41%) | 13 (27%) | 18 (37%) |
| Femmes : 49 (100%) | Mixte sèche : 22 (45%) | | | Utilisateurs réguliers : 14 (29%) |
| Hommes : 0 (0%) | Sèche : 21 (43%) | | | |
| Age moyen : 30 | Très Sèche : 2 (4%) | | | |
| Age minimum : 24 | | | | |
| Age maximum : 36 | | | | |

Tous ces volontaires présentaient des rides / ridules ainsi qu'un manque d'éclat et d'élasticité au niveau du visage.

Sur le plan des performances, l'ensemble des résultats figure dans le tableau 8 ci-dessous :

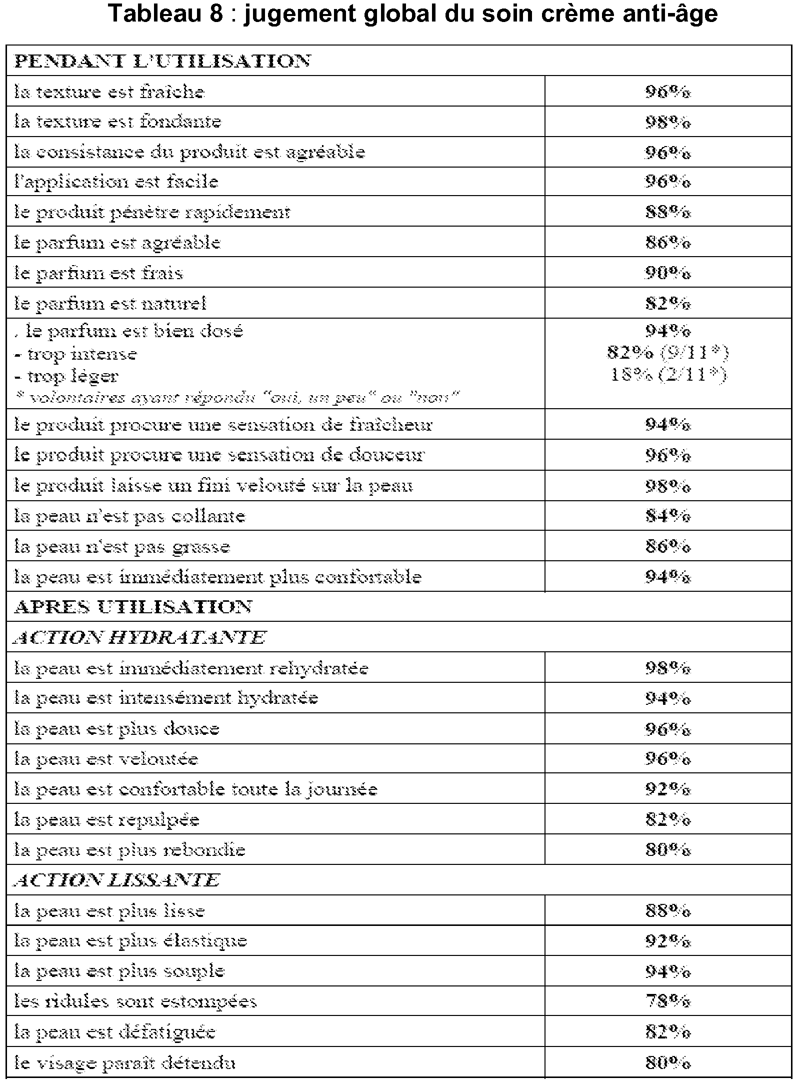

Un jugement positif en tant que "soin visage anti-âge" et appréciation favorable des volontaires pour les critères suivants (en % des volontaires interrogés - réponses "oui tout à fait" / "oui" / "oui un peu").

Après 4 semaines d'utilisation, les actions revendiquées sur le complexe actif sont validées par les volontaires comme le montre le tableau 9 ci-dessous.

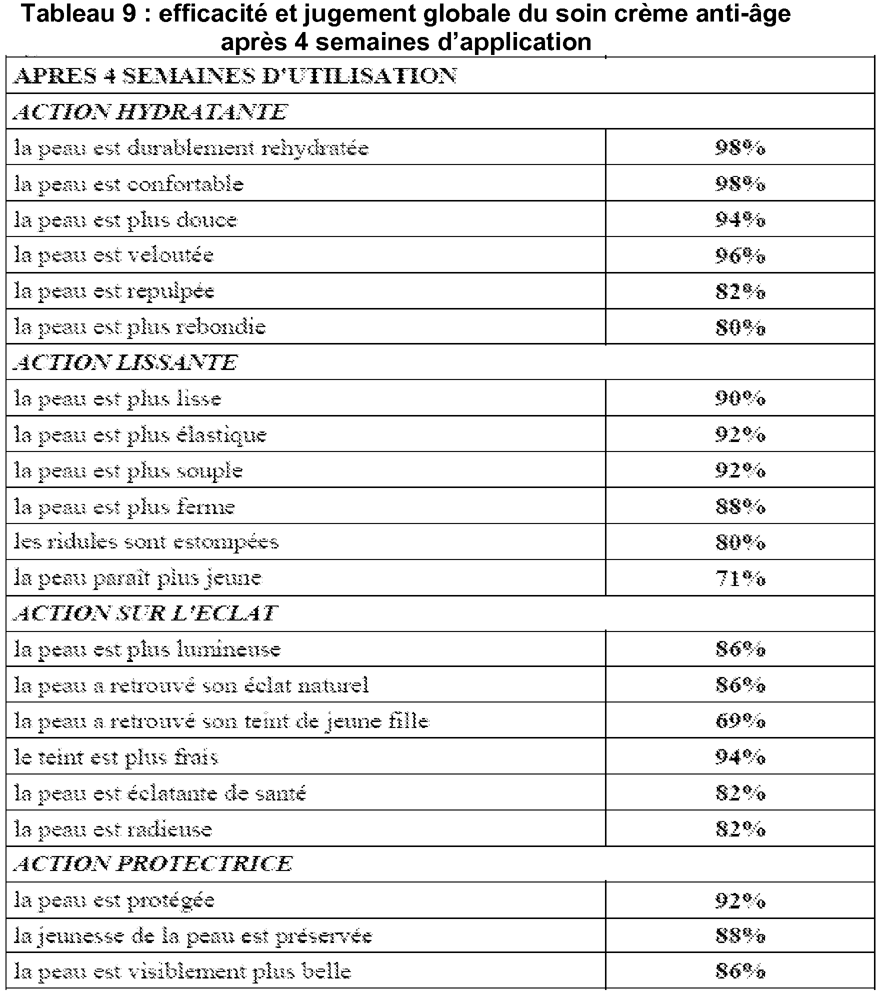

Le soin crème anti-âge hydratant, lissant, protecteur et actif sur l'éclat a donc été reconnu comme étant très efficace par les utilisateurs.

### Listes des références

[1] RAMEAU JC., MANSION D., DUME G., 1989. Flore forestière française. Tome 1 plaine et colline. Ed. IDF Institut pour le Developpement Forestier. 1785 p. (841).
[2] THEVENIN Thierry, 2008. Le chemin des herbes. Les plantes sauvages, connaître, cueillir et utiliser. ED. Lucien Souny. 331 p.
[3] FOURNIER, P., 1948. Encyclopédie biologique. Le livre des plantes médicinales et vénéneuses de France. 3 tomes.Tome I, 448 p. Paul Lechevalier editeur, Paris. (p. 93-97).
[4] Terre de semences « Pour la protection de la biodiversité » 1996. 124p. 03210 Saint-Menoux.
[5] Natural medicine: the genus Angelica. Sarker SD, Nahar L. Curr Med Chem. 2004 Jun;11(11):1479-500.
[6] Antitumour activity of Angelica archangelica leaf extract. Sigurdsson S, Ogmundsdottir HM, Hallgrimsson J, Gudbjarnason S. In Vivo. 2005 Jan-Feb;19(1):191-4.
[7] Hepatoprotective effect of Angelica archangelica in chronically ethanol-treated mice. Yeh ML, Liu CF, Huang CL, Huang TC. Pharmacology. 2003 Jun; 68(2):70-3.
[8] 14-Methylpentadecano-15-lactone (muscolide): a new macrocyclic lactone from the oil of Angelica archangelica L. Lopes D, Strobl H, Kolodziejczyk P. Chem Biodivers. 2004 Dec;1(12):1880-7.
[9] Les aquaporines. Laszlo, Pierre In La Science au présent 2009. Ed. Encyclopaedia Universalis, 2009.
[10] Occurrence of N-phenylpropenoyl-L-amino acid amides in différent herbal drugs and their influence on human keratinocytes, on human liver cells and on adhesion of Helicobacter pylori to the human stomach. Hensel A, Deters AM, Müller G, Stark T, Wittschier N, Hofmann T. Planta Med. 2007 Feb;73(2):142-50.
[11] Sharma Om P., Bhat T.K., DPPH antioxidant assay revisited. Food chemistry 2009, 113 (4), 1202.

## Revendications

1. Composition cosmétique comprenant un extrait d'Angelica archangelica en tant que principe actif anti-âge dans laquelle l'extrait d'Angelica archangelica est un mélange d'extrait aqueux et d'extrait huileux.

2. Composition selon la revendication 1, dans laquelle l'extrait aqueux est une eau d'Angelica archangelica.

3. Composition selon la revendication 1ou 2, dans laquelle l'extrait aqueux d'Angélique est présent dans la composition à une concentration de 0,01 à 10% en poids par rapport au poids total de ladite composition.

4. Composition selon l'une quelconque des revendications 1 à 3, dans laquelle l'extrait huileux est une huile essentielle de racine d'Angelica archangelica.

5. Composition selon la revendication 4, dans laquelle l'huile essentielle est présente dans la composition à une concentration de 0,001 à 0,8% en poids par rapport au poids total de ladite composition.

6. Composition selon l'une quelconque des revendications 1 à 5, ladite composition étant sous une forme choisie dans le groupe comprenant une crème, un lait, une lotion, un gel, un sérum, un onguent.

7. Procédé de soin cosmétique anti-âge comprenant une étape d'application sur la peau d'un extrait d'Angélique ou d'une composition cosmétique telle que définie dans l'une quelconque des revendications 1 à 6.

## Patentansprüche

1. Kosmetische Zusammensetzung, umfassend einen Angelica archangelica-Extrakt als Anti-Aging-Wirkstoff, wobei der Extrakt von Angelica archangelica eine Mischung aus wässrigem Extrakt und öligem Extrakt ist.

2. Zusammensetzung nach Anspruch 1, wobei der wässrige Extrakt ein Wasser von Angelica archangelica ist.

3. Zusammensetzung nach Anspruch 1 oder 2, wobei der wässerige Angelica-Extrakt in der Zusammensetzung in einer Konzentration von 0,01 bis 10 Gew.%, bezogen auf das Gesamtgewicht der Zusammensetzung, vorhanden ist.

4. Zusammensetzung nach einem der Ansprüche 1 bis 3, wobei der ölige Extrakt ein ätherisches Öl der Wurzel von Angelica archangelica ist.

5. Zusammensetzung nach Anspruch 4, wobei das ätherische Öl in der Zusammensetzung in einer Konzentration von 0,001 bis 0,8 Gew.%, bezogen auf das Gesamtgewicht der Zusammensetzung, vorhanden ist.

6. Zusammensetzung nach einem der Ansprüche 1 bis 5, wobei die Zusammensetzung in einer Form vorliegt, die ausgewählt ist aus der Gruppe bestehend aus einer Creme, einer Milch, einer Lotion, einem Gel, einem Serum, einer Salbe.

7. Kosmetisches Anti-Aging-Pflegeverfahren, umfassend einen Schritt des Auftragens auf die Haut eines Angelica-Extrakts oder einer kosmetischen Zusammensetzung, wie in einem der Ansprüche 1 bis 6 definiert.

## Claims

1. Cosmetic composition comprising an extract of Angelica archangelica as anti-ageing active ingredient, wherein the extract of Angelica archangelica is a mixture of aqueous extract and oily extract.

2. Composition according to Claim 1, wherein the aqueous extract is an Angelica archangelica water.

3. Composition according to Claim 1 or 2, wherein the aqueous angelica extract is present in the composition at a concentration from 0.01 to 10% by weight relative to the total weight of said composition.

4. Composition according to any one of Claims 1 to 3, wherein the oily extract is an essential oil of Angelica archangelica root.

5. Composition according to Claim 4, wherein the essential oil is present in the composition at a concentration from 0.001 to 0.8% by weight relative to the total weight of said composition.

6. Composition according to any one of Claims 1 to 5, said composition being in a form selected from the group consisting of a cream, a milk, a lotion, a gel, a serum or an ointment.

7. Cosmetic anti-ageing care process, comprising a step of applying an angelica extract or a cosmetic composition as defined in any one of Claims 1 to 6 to the skin.
